# EUROPEAN PATENT SPECIFICATION

(11) **EP 1 267 727 B1**
(45) Date of publication and mention of the grant of the patent: **11.05.2005**
(21) Application number: 01914245.4
(22) Date of filing: 02.03.2001
(51) Int. Cl.: A61B 17/00

(54) **CONTAINER LINK AND CONTAINER FOR STORAGE OF HAIR GRAFTS**
BEHÄLTERVERBINDUNG UND BEHÄLTER ZUR AUFBEWAHRUNG VON HAARIMPLANTATEN
RECEPTACLE A ELEMENTS DE CONNEXION ET RECEPTACLE POUR STOCKAGE DE GREFFON CAPILLAIRE

(30) Priority: 02.03.2000 NO 20001060
(43) Date of publication of application: 02.01.2003
(73) Proprietor: Hair Line AS, 1807 Askim (NO)
(72) Inventor: Werner, Per Gunnar, 1454 Fagerstrand (NO)
(74) Representative: Johansson, Lars E.
(86) International application number: PCT/NO2001/000083
(87) International publication number: WO 2001/064111

(56) References cited:
- FR-A1- 2 738 145
- US-A- 5 643 308
- US-A- 5 951 572

## Description

The invention relates to a container link for use in connection with drill-out, excision and implantation of hair grafts, wherein the link comprises a receptacle with a substantially cylindrical cavity for reception and storage of a graft. The invention also involves a container comprising such links.

Known methods of hair transplantation are characterized in that prior to implantation, the time-consuming and laborious task has to be performed of excising grafts from a piece of skin which has been surgically removed from an area with abundant hair growth at the nape of the patient's neck. The piece of skin is divided up manually by means of small knives into very thin strips, each containing 1-3 hair roots and placed in antiseptic liquid in dishes or on a moistened cloth. The hair roots must not be damaged during the splitting of the skin. Since the direction of the hair follicles varies from patient to patient and within one and the same piece of skin, great care must be taken in performing the cutting operation. The graft implantation is performed by the doctor lifting the grafts from the dish or the cloth with forceps and inserting them in predrilled holes in the skin of the head, whereupon the skin of the head is pressed together to prevent the grafts from being expelled by the bloodstream. Both the excision of the grafts and their implantation in the skin of the head take a long time. A negative consequence of this is that a long period elapses from the time when the blood supply to the hair roots is interrupted until the graft can again receive nourishment from the blood. This results in impairment of the hair roots, a long regrowth time and that some of the hair roots die during the process.

Newer methods for implanting hair roots employ devices both for drilling out and excising the donor skin as well as for implanting the grafts at the receiver location. The object of the present invention is to rationalise the total process by reducing the manual work involved and shortening the time from the removal at the donor location until the implantation at the receiver location, thereby substantially increasing the regrowth percentage and reducing the operating costs.

In order to achieve the most continuous process possible from the removal of the grafts from the donor location until the implantation at the receiver location, the method described herein employs a container with a large number of compartments for reception and short-term storage of grafts, which container may advantageously be able to act jointly with a guiding and advancing mechanism which is arranged both in the drill-out apparatus, the excision apparatus and in the implantation apparatus. When the container is filled with grafts from the drill-out apparatus or from the excision apparatus, the container is removed from the apparatus concerned and inserted in the implantation apparatus, whereupon the grafts are inserted mechanically in the receiver skin. Similar containers are previously known from inter alia FR-A-2.738.145 and US-A-5.951.572.

FR-A-2.738.145 describes a container for grafts consisting of tubular parts mounted beside one another together with a beam which provides stepwise advancement of the container to an implantation tool. The tubular parts are fixed together, thus making it impossible to remove empty tubular parts from the container.

US-A-5.951.572 discloses an apparatus for hair implantation, where the apparatus comprises a container. The container consists of cylindrical cells. In order to keep the grafts in place, the cells are extended in the longitudinal direction. This solution is suitable in the cases described where all the grafts are supplied to the cells by hand. When grafts are inserted in the receiving borings manually, there is no need to be able to remove an empty cell, as is the case when the excision or drill-out of the grafts and the insertion thereof in the cells are performed mechanically.

As stated above, in the prior art there is no possibility of removing a container compartment if it should be noted that this container compartment is empty, i.e. where there has been a failure to drill out and insert the graft in the container compartment. If a container is advanced to the implantation apparatus without all the container compartments having been filled, this will harm the patient, since holes will be made in the skin of his head which are not filled with hair roots.

The object of the invention is to solve the above-mentioned problems as well as to remedy other drawbacks in the prior art. These objects are achieved by means of a container link for use in connection with drill-out, excision and implantation of hair grafts, wherein the link comprises a receptacle with a substantially cylindrical cavity for reception and storage of a graft. The link according to the invention is characterized in that it is equipped with complementary connecting devices on opposite sides of the receptacle, the connecting devices being designed for releasable connection with corresponding connecting devices. The invention also comprises a container for receiving grafts, wherein the container according to the invention comprises individual receiving locations disposed beside one another and characterized in that the links are releasably interconnected by means of connecting devices.

In a preferred embodiment of the invention the connecting devices are composed of latch pins and holes for receiving the latch pins.
In a specially preferred embodiment of the invention the receptacle is provided with ridges which project from the sides of the receptacle into the cavity, and which extend at least along a part of the length of the receptacle, for centring and securing of the graft. The ridges are preferably uniformly distributed in the circumference of the cavity.

The container link according to the invention is preferably made of thermoplastic and by injection moulding.

The links are intended mainly for use together with devices for removal and implantation of grafts, and are therefore equipped in a preferred embodiment with an orientation fin to ensure correct positioning relative to such devices.

In order to facilitate the connection of the links, the container link may also be equipped with guide ridges for guiding the links relative to one another during the connection process.

In a particularly simple and practical embodiment the container link comprises a toothed part for interaction with a rack or a pinio, and the receptacle is preferably mounted in the toothed part. This results in savings with regard to space and material.

Preferred embodiments of the container according to the invention comprise links which have one or more of the above-mentioned features.

The invention will now be described in more detail with reference to the drawings, in which:
figs. 1a and 1b illustrate a container 1 for short-term storage of grafts,
figs. 2a and 2b illustrate a variant of the container illustrated in fig. 1,
figs. 3a, 3b and 3c illustrate a variant of the container illustrated in fig. 2,
figs. 4a, 4b, 4c and 4d illustrate a link in a preferred embodiment of the container according to the invention, and
figs. 5a and 5b illustrate several links as illustrated in fig. 4 assembled to form a container.
Fig. 6 illustrates several links connected together to form a container.

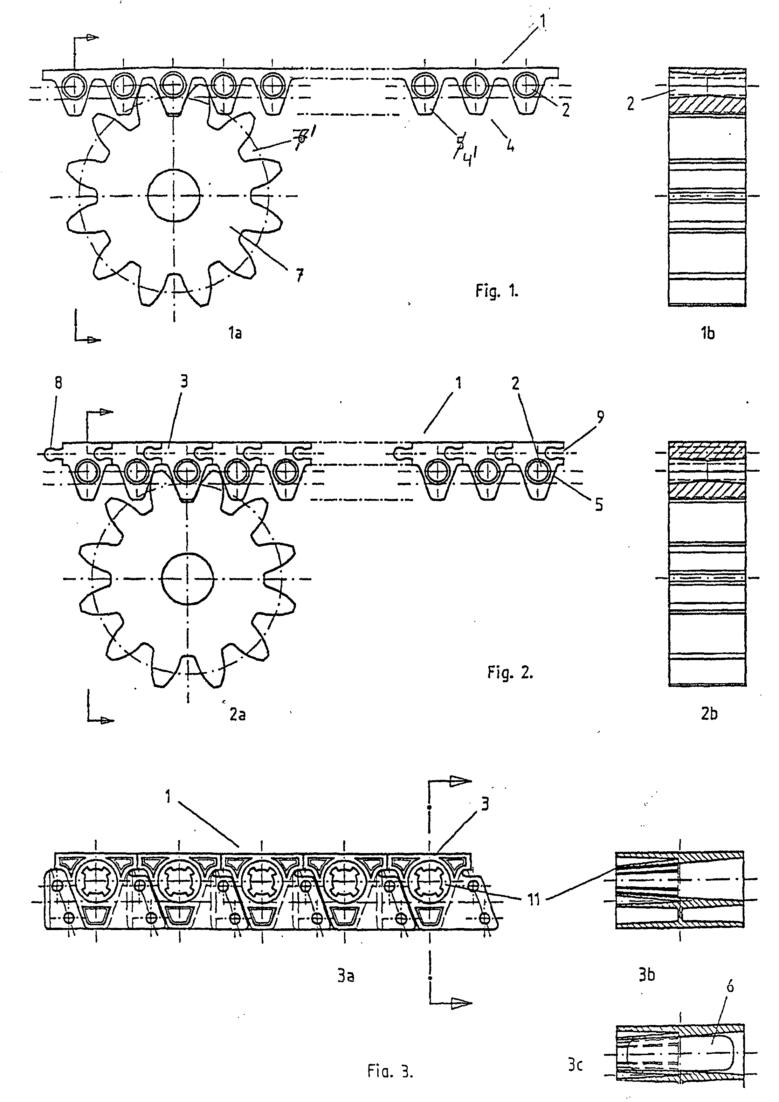

Fig. 1 illustrates a container 1 for storing a number of hair grafts, e.g. 50, where each individual graft is stored in a receptacle 5. The receptacle comprises an almost cylindrical cavity 2, also called a receiving compartment. The container 1 is arranged to be capable of being fed through a drill-out apparatus and an excision apparatus respectively, and to receive and secure the drilled and excised grafts respectively, and subsequently to be able to deliver the grafts to an implantation apparatus. The advancement operation is performed stepwise by means of a pawl device or a rack/toothed gear. A preferred embodiment which provides accurate positioning and which when required can feed the container both forwards and backwards is illustrated in the figure. On the underside of the container is a toothed rack 4 with a tooth 4' for each cavity 2 which is engaged with a toothed opening 7' in a ratchet wheel 7 driven by a step motor or a servomotor with an associated precise control device. The container 1 is arranged in such a manner that the individual receiving compartments 2 can be positioned exactly in alignment with the device drilling or excising the graft and the device inserting the graft under the skin of the patient's head. The grafts can thereby be pushed in a simple manner from the drill-out or excision device directly into the receiving compartments. When the container 1 is filled, it can be moved to the implantation apparatus, which is similarly equipped to be capable of feeding the container forwards and, if so desired, backwards. The grafts are inserted under the skin of the patient's head by means of the implantation apparatus.

The container illustrated in fig. 1, however, is not suitable to be moulded in thermoplastic. Firstly, a long item of this type will require a very expensive tool. Secondly, the material shrinkage in the hardening phase will result in changes in shape which will make the container unsuitable for the purpose.

The item, however, can be milled from a billet of a stable plastic material. However, this will be very expensive and impractical, since the containers will be unable to be reused for hygienic reasons.

Figures 2a and 2b illustrate a first embodiment of the invention. Here a container 1 is illustrated, which is divided up into connectable links 3, where each link 3 comprises a receptacle 5 which in this preferred embodiment corresponds to a tooth and a container compartment 2 and where each link 3 has complementary connecting devices 8 and 9 on opposite sides of the receptacle 5. By means of the connecting devices 8 and 9 the links 3 can be assembled to form a container 1. The links 3 can easily be disconnected in cases where a container compartment 2 is observed which does not contain a graft.
The container links 3 may be moulded in thermoplastic, and they can be mounted manually or mechanically. Furthermore, links 3 with empty container compartments 2 can be removed before the container in inserted in the implantation apparatus. However, items with so much material will require an unduly large amount of material, in addition to which they will be time-consuming and thereby expensive to mould. All things considered, an embodiment of this kind is not an optimal solution.

Figs. 3a, 3b and 3c illustrate a second embodiment of the container according to the invention, composed of a number of container links 3, which are suitable for injection moulding, almost all superfluous material having been removed by means of recesses 10 hollowed out from both sides. The material is of almost uniform thickness all over, and irregularities as a result of unequal shrinkage are almost eliminated. The container 1 is still suitable for use together with a pinio, since the links 3 are in the form of teeth.

In the receptacle 5, moreover, the container link 3 is equipped with a number of ridges 11 projecting from the sides of the receptacle 5 into the cavity 2, 4 such ridge being illustrated in the drawing, extending at least along a part of the length of the receptacle 5 and to some extent converging. It will, of course, be possible to vary the number of ridges 11 depending on the yield strength of the link material. The tasks of the ridges 11 are to guide the little graft 6 so that, regardless of variations in size and consistency, it is located in the middle of the cavity 2, and further to secure the graft 6 to prevent it from returning along with the ejector pin. The graft 6, which is sticky, will easily adhere to the ejector pin, and a design of the container compartment like that in the prior art can give the graft the opportunity to return along with the ejector pin. A further task of the ridges or grippers 11 is to exert a limited pressure on the graft 6, thus avoiding damage to the hair roots as a result of hard compression.

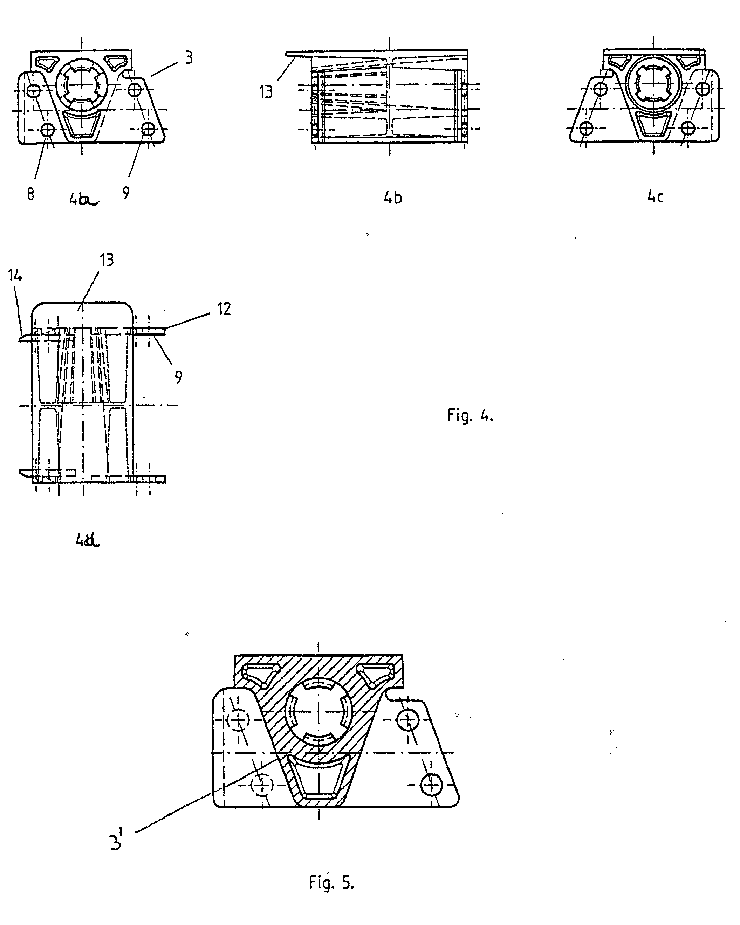

Figs. 4a, 4b, 4c and 4d illustrate a preferred embodiment of a container link 3. In addition to the above-described characteristics of the links 3, the connecting details are illustrated here, viz. a preferred embodiment of the connecting devices in the form of pins 8, which may have a slanting front rim for easier insertion into holes 9. The pins 8 and the holes 9 are preferably arranged on guide ridges 12. The figure also illustrates an orientation fin 13 which in a preferred embodiment projects on one side of the link 3 and is employed for preventing the container 1 from being inserted the wrong way into the drill-out and excision machines and the implantation apparatus. In the inlet and outlet channels of the above-mentioned apparatuses, there will be grooves on one side corresponding to the projecting orientation fin. Mistakes in installation will thereby be effectively prevented.
In order to further facilitate the connection process, an entry rim 14 may be provided to intercept and guide the guide ridges 12 during connection.
The above-mentioned orientation fin 13 may also be employed in the automatic sorting of the container links 3 prior to the automatic connection of a number of links 3 to form a complete container 1. The fin may be designed in many different ways, according to what is most beneficial for sorting and correctly inserting the container in the apparatuses.

Fig. 5 illustrates a container link 3, as described above, which is intersected near the middle. The container link 3 is in the form of a tooth, and the tooth flanks 3' are at a 20° pressure angle according to international standard. Another pressure angle may be chosen, if this is found to be expedient.

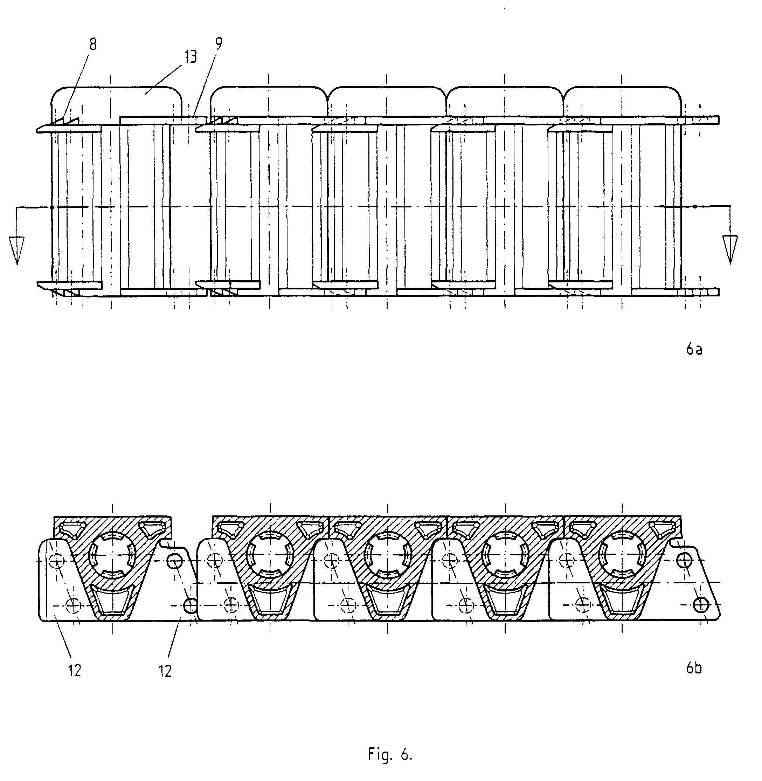

Figure 6 illustrates several links 3 connected together to form a container. The links 3 are equipped with pins 8 and holes 9 arranged in guide ridges 12 projecting from the receptacle. The guide ridges 12 are also used as guiding means for guiding the links 3 into the correct position relative to one another.

## Claims

1. A container link (3) for use in connection with drill-out, excision and implantation of hair grafts (6), wherein the link (3) comprises a receptacle (5) with a substantially cylindrical cavity (2) for reception and storage of a graft,
**characterized in that** the link (3) is equipped with complementary connecting devices (8,9) on opposite sides of the receptacle, the connecting devices being designed for releasable connection with corresponding connecting devices.

2. A container link (3) according to claim 1,
**characterized in that** the connecting devices are composed of latch pins and holes for reception of the latch pins.

3. A container link (3) according to one of the preceding claims,
**characterized in that** the receptacle (5) is provided with ridges (11) projecting from the sides of the receptacle (5) into the cavity (2), and extending at least along a part of the length of the receptacle (5) for centring and securing the graft (6).

4. A container link (3) according to claim 3,
**characterized in that** the ridges are uniformly distributed in the circumference of the cavity (2).

5. A container link (3) according to one of the preceding claims,
**characterized in that** it is made of thermoplastic.

6. A container link (3) according to one of the preceding claims,
**characterized in that** it is made by injection moulding.

7. A container link (3) according to one of the preceding claims,
**characterized in that** it is equipped with an orientation fin (13) in order to ensure correct positioning relative to a removal and/or implantation device for grafts.

8. A container link (3) according to one of the preceding claims,
**characterized in that** it is equipped with guide ridges (12) for guiding the links (3) relative to one another during the connecting process.

9. A container link (3) according to one of the preceding claims,
**characterized in that** it comprises a toothed part (4') for interaction with a rack or a pinion.

10. A container link (3), according to one of the preceding claims,
**characterized in that** the receptacle (5) is provided in the toothed part (4').

11. A container for use in connection with drill-out, excision and implantation of hair grafts, wherein the container comprises container links (3) with a receptacle (5) with a substantially cylindrical cavity (2) for reception and storage of a graft (6),
**characterized in that** the links (3) are releasably interconnected by means of connecting devices (8,9).

12. A container according to claim 11,
**characterized in that** it comprises links (3) according to one of the claims 2-8.

## Patentansprüche

1. Behälterverbindungsglied (3) zur Verwendung in Verbindung mit einem Ausbohren, Ausschneiden und Implantieren von Haartransplantaten (6),
wobei das Verbindungsglied (3) ein Aufnahmeelement (5) mit einem im Wesentlichen zylindrischen Hohlraum (2) zur Aufnahme und Lagerung eines Transplantats umfasst,
**dadurch gekennzeichnet, dass**
das Verbindungsglied (3) auf entgegengesetzten Seiten des Aufnahmeelements mit komplementären Verbindungseinrichtungen (8, 9) ausgestattet ist, wobei die Verbindungseinrichtungen für eine lösbare Verbindung mit entsprechenden Verbindungseinrichtungen ausgelegt sind.

2. Behälterverbindungsglied (3) nach Anspruch 1,
**dadurch gekennzeichnet, dass**
die Verbindungseinrichtungen aus Einrastzapfen und Öffnungen zur Aufnahme der Einrastzapfen bestehen.

3. Behälterverbindungsglied (3) nach einem der vorstehenden Ansprüche,
**dadurch gekennzeichnet, dass**
das Aufnahmeelement (5) mit Stegen (11) versehen ist, die von den Seiten des Aufnahmeelements (5) in den Hohlraum (2) vorstehen und sich zumindest entlang eines Teils der Länge des Aufnahmeelements (5) erstrecken, um das Transplantat (6) zu zentrieren und festzuhalten.

4. Behälterverbindungsglied (3) nach Anspruch 3,
**dadurch gekennzeichnet, dass**
die Stege im Umfang des Hohlraums (2) gleichmäßig verteilt sind.

5. Behälterverbindungsglied (3) nach einem der vorstehenden Ansprüche,
**dadurch gekennzeichnet, dass**
es aus thermoplastischem Kunststoff besteht.

6. Behälterverbindungsglied (3) nach einem der vorstehenden Ansprüche,
**dadurch gekennzeichnet, dass**
es durch Spritzgießen hergestellt wird.

7. Behälterverbindungsglied (3) nach einem der vorstehenden Ansprüche,
**dadurch gekennzeichnet, dass**
es mit einem Ausrichtungssteg (13) ausgestattet ist, um die richtige Positionierung bezüglich einer Entnahme- und/oder Implantationseinrichtung für Transplantate sicherzustellen.

8. Behälterverbindungsglied (3) nach einem der vorstehenden Ansprüche,
**dadurch gekennzeichnet, dass**
es mit Führungsrippen (12) ausgestattet ist, um die Verbindungsglieder (3) während des Verbindungsvorgangs in Bezug aufeinander zu führen.

9. Behälterverbindungsglied (3) nach einem der vorstehenden Ansprüche,
**dadurch gekennzeichnet, dass**
es ein gezahntes Teil (4') für ein Zusammenwirken mit einer Zahnstange oder einem Zahnrad umfasst.

10. Behälterverbindungsglied (3) nach einem der vorstehenden Ansprüche,
**dadurch gekennzeichnet, dass**
das Aufnahmeelement (5) im gezahnten Teil (4') vorgesehen ist.

11. Behälter zur Verwendung in Verbindung mit einem Ausbohren, Ausschneiden und Implantieren von Haartransplantaten,
wobei der Behälter Behälterverbindungsglieder (3) mit einem Aufnahmeelement (5) mit einem im Wesentlichen zylindrischen Hohlraum (2) zur Aufnahme und Lagerung eines Transplantats (6) umfasst,
**dadurch gekennzeichnet, dass**
die Verbindungsglieder (3) mittels Verbindungseinrichtungen (8, 9) lösbar miteinander verbunden sind.

12. Behälter nach Anspruch 11,
**dadurch gekennzeichnet, dass**
er Verbindungsglieder (3) nach einem der Ansprüche 2 bis 8 umfasst.

## Revendications

1. Elément de connexion de récipient (3) destiné à être utilisé en liaison avec la perforation, l'excision et l'implantation de greffons capillaires (6), dans lequel l'élément de connexion (3) comprend un réceptacle (5) avec une cavité (2) sensiblement cylindrique destiné à la réception et au stockage d'un greffon, **caractérisé en ce que** l'élément de connexion (3) est équipé de dispositifs de raccordement complémentaires (8, 9) sur les côtés opposés du réceptacle, les dispositifs de raccordement étant conçus en vue du raccordement amovible aux dispositifs de raccordement correspondants.

2. Elément de connexion de récipient (3) selon la revendication 1,
**caractérisé en ce que** les dispositifs de raccordement sont composés de goupilles de verrouillage et de trous destinés à la réception des goupilles de verrouillage.

3. Elément de connexion de récipient (3) selon l'une quelconque des revendications précédentes,
**caractérisé en ce que** le réceptacle (5) est pourvu de saillies (11) se projetant depuis les côtés du réceptacle (5) à l'intérieur de la cavité (2), et s'étendant au moins le long d'une partie de la longueur du réceptacle (5) pour centrer et fixer le greffon (6).

4. Elément de connexion de récipient (3) selon la revendication 3,
**caractérisé en ce que** les saillies sont distribuées uniformément dans la circonférence de la cavité (2).

5. Elément de connexion de récipient (3) selon l'une quelconque des revendications précédentes,
**caractérisé en ce que** qu'il est fabriqué en matière thermoplastique.

6. Elément de connexion de récipient (3) selon l'une quelconque des revendications précédentes,
**caractérisé en ce qu'**il est fabriqué par moulage par injection.

7. Elément de connexion de récipient (3) selon l'une quelconque des revendications précédentes,
**caractérisé en ce qu'**il est équipé d'une ailette d'orientation (13) afin de garantir un positionnement correct par rapport à un dispositif de retrait et/ou d'implantation de greffons.

8. Elément de connexion de récipient (3) selon l'une quelconque des revendications précédentes,
**caractérisé en ce qu'**il est équipé de saillies de guidage (12) destinées à guider les éléments de connexion (3) les uns par rapport aux autres pendant le processus de raccordement.

9. Elément de connexion de récipient (3) selon l'une quelconque des revendications précédentes,
**caractérisé en ce qu'**il comprend une partie dentée (4') destinée à interagir avec un râtelier ou un pignon.

10. Elément de connexion de récipient (3) selon l'une quelconque des revendications précédentes,
**caractérisé en ce que** le réceptacle (5) est pourvu de la partie dentée (4').

11. Récipient destiné à être utilisé en liaison avec la perforation, l'excision et l'implantation de greffons capillaires, dans lequel le récipient comprend des éléments de connexion de récipient (3) avec un réceptacle (5) présentant une cavité (2) sensiblement cylindrique destiné à la réception et au stockage d'un greffon (6),
**caractérisé en ce que** les éléments de connexion (3) sont interconnectés de façon amovible au moyen de dispositifs de raccordement (8, 9).

12. Récipient selon la revendication 11,
**caractérisé en ce qu'**il comprend des éléments de raccordement (3) selon l'une des revendications 2 à 8.
